(19) Europäisches Patentamt
European Patent Office
Office européen des brevets

(11) Publication number: **0 235 714 B1**

(12) **EUROPEAN PATENT SPECIFICATION**

(45) Date of publication of patent specification: **29.08.90**

(51) Int. Cl.$^5$: **C 07 C 25/24,** C 07 B 35/06

(21) Application number: **87102510.2**

(22) Date of filing: **18.10.83**

(60) Publication number of the earlier application in accordance with Art. 76 EPC: **0 110 536**

# (54) Process for the preparation of compounds with a double carbon to carbon bond by dehydration of the corresponding alcohol.

(30) Priority: **10.11.82 GB 8232083**
**01.02.83 GB 8302751**

(43) Date of publication of application:
**09.09.87 Bulletin 87/37**

(45) Publication of the grant of the patent:
**29.08.90 Bulletin 90/35**

(84) Designated Contracting States:
**BE CH DE FR GB IT LI NL**

(56) References cited:
**US-A-4 049 736**
**US-A-4 150 059**
**HOUBEN-WEYL: "Methoden der organischen Chemie", vol. V/1b, "Alkene, cycloalkene, arylalkene (mit isolierten C=C-Doppelbindungen)", 1972, pages 44-62, Georg Thieme Verlag, Stuttgart, DE**
**BAS; AMSTERDAM VOLUME 88, NUMBER 12, December 1969, pages 1424-1436**
**HOUBEN-WEYL: "Methoden der organischen Chemie" vol V/lb, pages 62, 82, 103, 104.**

(73) Proprietor: **IMPERIAL CHEMICAL INDUSTRIES PLC**
**Imperial Chemical House, Millbank**
**London SW1P 3JF (GB)**

(72) Inventor: **Jones, Raymond Vincent Heavon**
**26 Clarendon Road**
**Linlithgow West Lothian Scotland (GB)**
Inventor: **Fleming, Ian George Cameron**
**56 Newmains Road**
**Kirkliston Scotland (GB)**
Inventor: **Ewins, Robert Comgall**
**23 Tantallon Drive Carron Falkirk**
**Stirlingshire FK2 8DJ Scotland (GB)**
Inventor: **Jones, John David**
**57 Keats Road**
**Greenmount Bury BL8 4EP (GB)**

(74) Representative: **Houghton, Malcolm John et al**
**Imperial Chemical Industries PLC Legal Department: Patents PO Box 6**
**Welwyn Garden City Herts, AL7 1HD (GB)**

**EP 0 235 714 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Courier Press, Leamington Spa, England.

**Description**

This invention relates to a process for the preparation of compounds with a double carbon to carbon bond, that is to say, a process for the preparation of certain substituted alkenes.

In Recueil Des Travaux Chimique Des Pays Bas, Amsterdam, Vol 88 No. 12, pages 1424—1436, the dehydration of 2-aryl-3-methyl-2-butanols in the presence of an aprotic medium is disclosed. In Houben-Weyl:Methoden der Organischen Chemie, Vol V/1b, pages 44—62, 82, 103 and 104, the use of alumina as an aprotic dehydrating agent is disclosed.

The alkenes of formula (II) below are described in European Patent Application No. 83306310.0 of the same effective date (European Patent Specification No. 0110536).

According to the present invention there is provided a process for the preparation of substituted alkenes of formula (II):

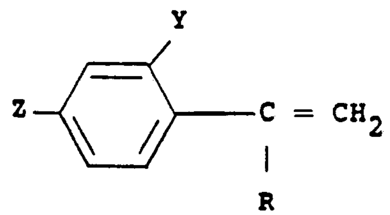

(II)

in which Y and Z are independently hydrogen, chloro or fluoro provided at least one of Y and Z is chloro or fluoro, and R is $C_3$ or $C_4$ alkyl; which comprises dehydrating an alcohol of formula (V):

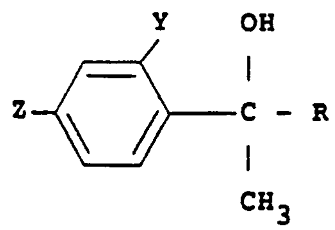

(V)

in which R, Y and Z have the meanings given above.

Dehydration may be carried out by methods known from the literature for such reactions, for example, by heating the alcohol of formula (V) in the presence of a catalytic amount of a strong acid such as *p*-toluene-sulphonic acid, preferably in the presence of an inert solvent, stirring the alcohol in a mixture of sulphuric and glacial acetic acids or heating it in refluxing dimethyl sulphoxide.

As well as forming the compound of formula (II) in the dehydration process, another alkene may also be formed except in the case when R is *tertiary* alkyl. This other alkene has the formula (VI):

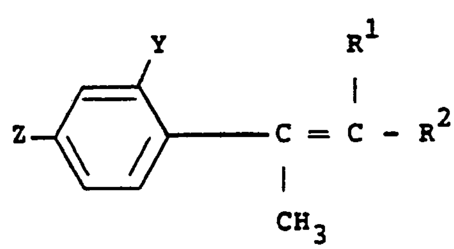

(VI)

in which $R^1$ and $R^2$ are independently H or alkyl and are related to R such that R is $—CHR^1R^2$.

For example, under certain acid conditions, the alcohol (2-(2,4-dichlorophenyl)-*n*-hexan-2-ol dehydrates to a mixture of 30% 2-(2,4-dichlorophenyl)-*n*-hex-1-ene and 70% 2-(2,4-dichlorophenyl)-*n*-hex-2-ene. The percentage of the hex-1-ene can be increased to 62 and that of the hex-2-ene reduced to 38 by conducting the dehydration in refluxing dimethyl sulphoxide.

In a preferred process for dehydrating the alcohol of formula (V), the alcohol of formula (V) is heated at a temperature of 100°C or above in the presence of an alumina catalyst. Suitably the process is carried out in the liquid phase at a temperature below the boiling point of the alcohol but sufficiently high to enable the dehydration to be completed in an acceptable time. For example, the alcohol 2-(2,4-dichlorophenyl)-*n*-hexan-2-ol can be dehydrated at a temperature of about 220°C in 4 to 5 hours in the presence of about a fifth of its weight of alumina.

Surprisingly, dehydration under these conditions favours more strongly formation of the alkene of formula (II). For example, 2-(2,4-dichlorophenyl)-*n*-hexan-2-ol is dehydrated to a mixture of the corresponding hex-1-ene and hex-2-ene in the ratio of approximately 82:18.

The alcohol of formula (V) may be obtained by a Grignard reaction between a methyl magnesium halide and a compound of formula (III):

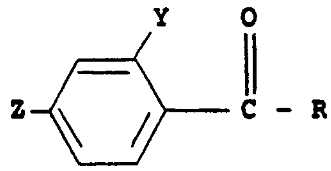

(III)

which may be obtained by Friedel-Crafts reaction between an acyl chloride of formula RCOCl and a benzene derivative of formula (IV):

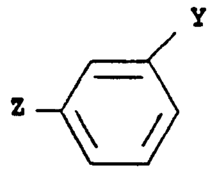

(IV)

in which R, Y and Z have the meanings stated above, in the presence of a Lewis acid such as aluminium chloride.

The substituted alkenes of formula (II) are useful intermediates for fungicides of formula (VII):

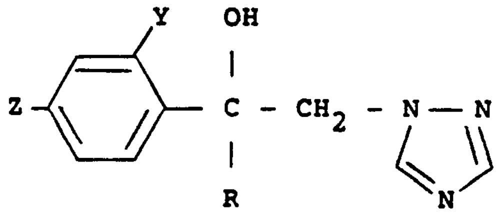

(VII)

in which R, Y and Z have the meanings stated above, which are described in, for example, European Patent Specification No. 48548 (European Application No. 81303753.8) and UK Patent Specification No. 2064520.

The invention is illustrated by the following Example in which parts and percentages are by weight.

Example 1

Dehydration of 2-(2,4-dichlorophenyl)-n-hexan-2-ol (A) to 2-(2,4-dichlorophenyl)-n-hex-1-ene (B) and 2-(2,4-dichlorophenyl)-n-hex-2-ene (C) using alumina

Alumina (Harshaw A13996R) catalyst (1 g) and 2-(2,4-dichlorophenyl)-*n*-hexan-2-ol (5 g) were stirred together and heated at approximately 220°C for 4½ hours under a blanket of nitrogen. Samples of the liquid component were taken periodically and analysed by gas chromatography. The results of the analyses are tabulated below.

After the 4½ hours, Carbon DY3 was added and the mixture filtered to give a clear yellow liquid.

| Sample | Time (hrs) | A(%) | B(%) | C(%) | Unknown (X) (%) | Mass Balance | Ratio of B:C | Ratio of B:C:X |
|---|---|---|---|---|---|---|---|---|
| 1 | ½ | 108.0 | 1.2 | 0.3 | 0.08 | 100+ | 80:20 | 76:19:5 |
| 2 | 1 | 93.0 | 9.8 | 2.0 | 1.1 | 100+ | 83:17 | 76:16:8 |
| 3 | 1½ | 70.5 | 21.4 | 4.4 | 2.3 | 98.6 | 83:17 | 76:16:8 |
| 4 | 2 | 36.3 | 45.8 | 9.3 | 4.8 | 96.2 | 83:17 | 76:16:8 |
| 5 | 3 | 12.8 | 61.5 | 12.8 | 6.4 | 93.5 | 83:17 | 76:16:8 |
| 6 | 4 | 0.6 | 69.9 | 15.1 | 7.5 | 93.1 | 82:18 | 76:16:8 |
| 7 | 4½+ cleaning | 0 | 69.8 | 15.1 | 7.5 | 92.4 | 82:18 | 76:16:8 |

$^1$H. N.M.R. analysis of the final sample indicated an isomer ratio (B:C) of 86:14.

Example 2

Dehydration of 2-(2,4-dichlorophenyl)-n-hexan-2-ol (A) to 2-(2,4-dichlorophenyl)-n-hex-1-ene (B) and 2-(2,4-dichlorophenyl)-n-hex-2-ene (C) using sulphuric/acetic acid

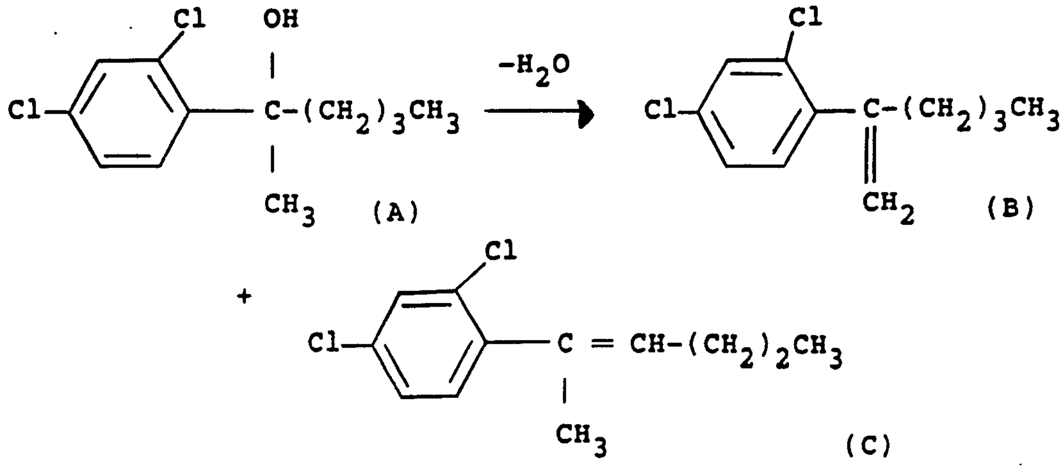


(A) (B) (C)

A mixture of 2-(2,4-dichlorophenyl)-hexan-2-ol (1.0 g; 4.05 m.mole) and 0.435M sulphuric acid in glacial acetic acid (20 ml) was stirred at ambient temperature (20—25°C) for 5 hours. The mixture was drowned into dilute sodium hydroxide solution and the product was extracted into carbon tetrachloride.

P.m.r. spectrum showed the product comprises about 34% mole 2-(2,4-dichlorophenyl)-hex-1-ene (B) and 66% mole 2-(2,4-dichlorophenyl)-hex-2-ene (C).

Example 3

Dehydration of 2-(2,4-dichlorophenyl)-n-hexan-2-ol (A) to 2-(2,4-dichlorophenyl)-n-hex-1-ene (B) and 2-(2,4-dichlorophenyl)-n-hex-2-ene (C) using dimethylsulphoxide

A solution of 2-(2,4-dichlorophenyl)hexan-2-ol (1.0 g; 4.05 m.mole) and dimethylsulphoxide-$d_6$ (5 ml) was held on reflux for 12 hours.

P.m.r. spectrum showed the product comprised about 62% mole 2-(2,4-dichlorophenyl)-hex-1-ene (B) and 38% mole 2-(2,4-dichlorophenyl)-hex-2-ene (C).

**Claims**

1. Process for the preparation of substituted alkenes of formula (II):

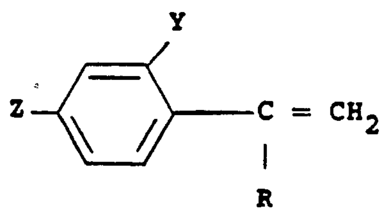

(II)

in which Y and Z are independently hydrogen, chloro or fluoro provided at least one of Y and Z is chloro or fluoro, and R is $C_3$ or $C_4$ alkyl; which comprises dehydrating an alcohol of formula (V):

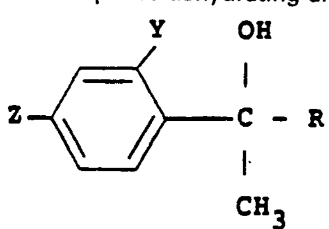

(V)

in which R, Y and Z have the meanings given above.

2. A process for the preparation of substituted alkenes according to claim 1 which comprises heating an alcohol of formula (V):

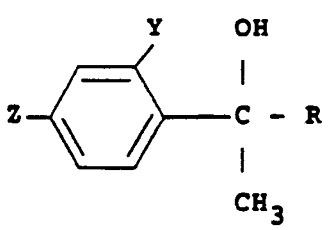

(V)

in which R, Y and Z have the meanings given in claim 1, at a temperature of 100°C or above in the presence of an alumina catalyst.

**Patentansprüche**

1. Verfahren zur Herstellung von substituierten Alkenen der Formel (II)

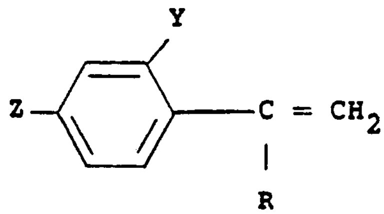

(II)

worin Y und Z unabhängig für Wasserstoff, Chloro oder Fluoro stehen, mit der Maßgabe, daß mindestens eines der Symbole Y und Z Chloro oder Fluoro bedeutet, und R für $C_3$- oder $C_4$-Alkyl steht, bei welchem ein Alkohol der Formel (V)

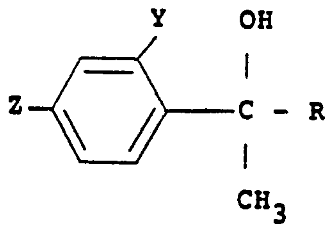

(V)

worin R, Y und Z die oben angegebenen Bedeutungen besitzen, dehydratisiert wird.

2. Verfahren zur Herstellung von substituierten Alkenen nach Anspruch 1, bei welchem ein Alkohol der Formel (V)

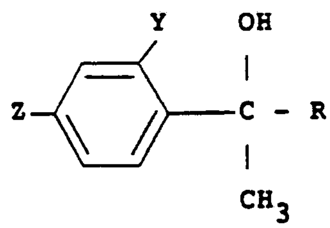

(V)

worin R, Y und Z die in Anspruch 1 angegebenen Bedeutungen besitzen, in Gegenwart eines Aluminiumoxid-Katalysators auf eine Temperatur von 100°C oder darüber erhitzt wird.

**Revendications**

1. Procédé de préparation d'alcènes substitués de formule (II):

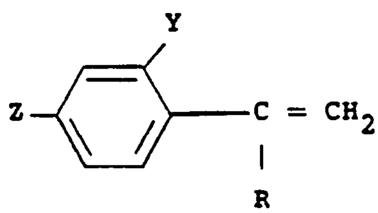

(II)

dans laquelle Y et Z représentent indépendamment l'hydrogène, des groupes chloro ou fluoro, sous réserve qu'au moins un des groupes Y et Z représente un groupe chloro ou fluoro, et R représente un groupe alkyle en $C_3$ ou $C_4$, qui consiste à déshydrater un alcool de formule (V):

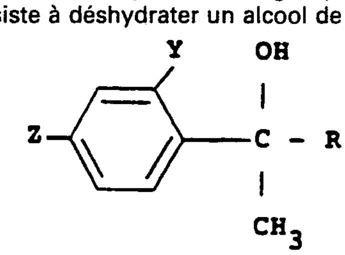

(V)

dans laquelle R, Y et Z répondent aux définitions précitées.

2. Procédé de préparation d'alcènes substitués suivant la revendication 1, qui consiste à chauffer un alcool de formule (V):

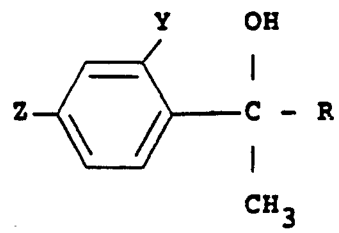

(V)

dans laquelle R, Y et Z répondent aux définitions mentionnées dans la revendication 1, à une température égale ou supérieure à 100°C en présence d'un catalyseur constitué d'alumine.